Europäisches Patentamt

European Patent Office

Office européen des brevets

(11) Numéro de publication: **0 322 262 B1**

(12) **FASCICULE DE BREVET EUROPEEN**

(45) Date de publication de fascicule du brevet: **13.01.93**

(51) Int. Cl.5: **C07K 13/00**, C07K 7/06, C12N 9/20, G01N 33/68, A61K 37/02

(21) Numéro de dépôt: **88402884.6**

(22) Date de dépôt: **17.11.88**

(54) **Protéine basique dénommée phospholipase A2 isolée de venin de serpent de la famille des élapidés et sa séquence en amino-acides, dérivés et fragments de ladite protéine, leur procédé d'obtention, compositions thérapeutiques et agents de diagnostic contenant ladite protéine et/ou ses dérivés et/ou ses fragments.**

(30) Priorité: **20.11.87 FR 8716096**

(43) Date de publication de la demande:
**28.06.89 Bulletin 89/26**

(45) Mention de la délivrance du brevet:
**13.01.93 Bulletin 93/02**

(84) Etats contractants désignés:
**CH DE GB LI NL**

(56) Documents cités:

**THE JOURNAL OF BIOCHEMISTRY, vol. 98, no. 2, août 1985, pages 289-303, The Japanese Biochemical Society; N. TAMIYA et al.: "Non-divergence theory of evolution: Sequence comparison of some proteins from snakes and bacteria"**

(73) Titulaire: **COMMISSARIAT A L'ENERGIE ATOMIQUE Etablissement de Caractère Scientifique Technique et Industriel**
**31/33, rue de la Fédération**
**F-75015 Paris(FR)**

(72) Inventeur: **Menez, André**
**109 avenue Claude Nicolas Ledoux Magny les Hameaux**
**F-78470 St Rémy Les Chevreuse(FR)**
Inventeur: **Chwetzoff, Serge**
**Le Coteau 1 rue de la Gruerie**
**F-91190 Gif Sur Yvette(FR)**

(74) Mandataire: **Ores, Irène et al**
**CABINET ORES 6, Avenue de Messine**
**F-75008 Paris(FR)**

EP 0 322 262 B1

Rank Xerox (UK) Business Services

TOXICON, vol. 19, 1981, pages 61-71, Perga- mon Press Ltd, GB; E. CONDREA et al.: "Effect of modification of one histidine resi- due on the enzymatic and pharmacological properties of a toxic phospholipase A2 from Naja Nigricollis snake venom and less toxic phospholipases A2 from Hemachatus Hae- machatus and Naja Naja Atra snake ve- noms"

LIFE SCIENCES, vol. 40, no. 16, 20 avril 1987, pages 1601-1607, Pergamon Journals Ltd., New York, US; A. CHAIM-MATYAS et al.: "Cytotoxic activity of various snake venoms on melanoma, B16F10 and chondrosarcoma"

PHARMACIA, septembre 1986, FPMG 50-01-329, pages 6-7, 17-18, Laboratory Sepa- ration Division, Uppsala, SE;" FPLC: Media and column guide - High performance sepa- ration of biomolecules"

EUROPEAN JOURNAL OF BIOCHEMISTRY, vol. 137, no. 3, 15 décembre 1983, pages 545-551, FEBS; M.J. DUFTON et al.: "Classification of phospholipases A2 accor- ding to sequence - Evolutionary and phar- macological implications"

TOXICON, vol. 24, no. 7, 1986, pages 679-693, Pergamon Journals Ltd, GB; K.R. SOONS et al.: "Effects of modification of tyrosines 3 and 62 (63) on enzymatic and toxicological properties of phospholipases A2 from Naja Nigricollis and Naja Naja Atra snake ve- noms"

**Description**

La présente invention est relative à une protéine basique isolée de venin de serpent de la famille des Elapidés, notamment d'un serpent Naja et plus particulièrement de Naja mossambica pallida et/ou de Naja nigricollis, dénommée phospholipase A2 (PLA2), aux fragments et dérivés de ladite protéine, à leurs procédés d'obtention, à des compositions thérapeutiques utilisables en médecine humaine et/ou vétérinaire, ainsi qu'à des agents de diagnostic contenant ladite protéine et/ou ses dérivés et/ou ses fragments.

Il existe à l'heure actuelle de nombreux agents antitumoraux, substances organiques ou inorganiques. Ces différents agents ont une action sur le noyau cellulaire, en particulier celui des cellules tumorales. Selon les substances, il existe plusieurs mécanismes d'action : soit lesdites substances interfèrent avec la biosynthèse des acides nucléiques et des protéines, soit elles interfèrent avec la duplication de l'A D N , soit elles agissent sur le fuseau mitotique. Un certain nombre d'auteurs ont mis en évidence ces différents mécanismes d'action (RALPH R.K. et al., TIBS, 1983, 212-214, LIPPARD, Science, 1982, 218, 4577, 1075-1082, FAUVAUDON, Biochimie, 1982, 64, 457-475).

Toutefois, les substances anticancéreuses n'exercent pas spécifiquement leur action sur les cellules cancereuses. Elles inhibent les divisions cellulaires des cellules saines comme des cellules cancéreuses : elles sont donc toxiques pour tous les tissus où les cellules se renouvellent rapidement. Les différentes substances antitumorales évoquées présentent donc un grand nombre d'effets indésirables chez l'homme.

L'importance des effets indésirables a conduit à rechercher de nouveaux agents antitumoraux, ayant un mode et un site d'action différents et ne présentant pas les effets indésirables tels que ceux rencontrés avec les substances antitumorales existantes.

Récemment, il a été démontré que certaines substances secrétées par des cellules de diverses lignées, telles que, notamment, les lymphocytes B et T, ainsi que les macrophages (Ruddle, Immunol. Today, 1987, 8, 5, 129-130) sont capables de tuer les cellules de certaines lignées tumorales. Les gènes (ADNc) codant pour ces substances ont été clonés (GRAY et al., Nature, 1984, 312, 721-724 et PENNICA et al., Nature, 1984, 312, 724-727). On nomme ces substances, facteurs de nécrose des tumeurs(TNF) et lymphotoxines (LT) ; il faut noter que le mécanisme d'action de ces substances est à l'heure actuelle, inconnu.

Un autre groupe de substances, est extrêmement intéressant ; il s'agit de substances présentes dans les venins de serpent ; en effet ceux-ci sont capables de tuer les cellules cancéreuses de nombreuses lignées (CHAIMMATYAS et al., Life Sciences, 1987, 40, 16, 1601-1607) ; les auteurs de cette publication ont en particulier montré que les venins de serpent présentent une activité cytotoxique vis à vis de certains mélanomes et chondrosarcomes, tant in vitro qu'in vivo et que, notamment, le venin de Naja nigricollis présente une forte activité cytotoxique.

Cette activité cytotoxique semble fondée sur un mécanisme de lyse membranaire des cellules concernées, totalement différent des mécanismes d'action des substances antitumorales de l'Art antérieur.

Le venin de cobra cracheur d'Afrique de l'Est, Naja nigricollis, comme le venin de la plupart des Elapidés, renferme trois classes de protéines toxiques : des toxines curarisantes, des cardiotoxines et des phospholipases.

La cardiotoxine, élément prépondérant du venin est pour une part, responsable de l'action cytotoxique ; cependant l'activité cytotoxique du venin total est nettement supérieure à celle de la cardiotoxine seule. En outre, l'action cytotoxique du venin est conservée en présence de calcium, un inhibiteur de l'activité cytotoxique des cardiotoxines. Un ou plusieurs facteurs cytotoxiques, autre que la cardiotoxine, existent donc dans le venin de Naja, en particulier de Naja nigricollis.

Les phospholipases sont classées en différents groupes, en fonction de leur létalité. Le premier groupe inclut des phospholipases A2 acides ou neutres, présentant une faible toxicité ; ces PLA2 assurent essentiellement la digestion des tissus phospholipidiques. Le second groupe est composé de PLA2 extrêmement toxiques ; ce sont, soit des molécules basiques monomères, soit des molécules contenant une sous-unité basique. On trouve ces composés dans de nombreux venins de serpents, ceux-ci provoquant une paralysie rapide des proies. Ils sont caractérisés par une capacité de blocage de la libération de l'acétylcholine au niveau des terminaisons présynaptiques et par d'autres effets incluant notamment une myotoxicité importante. Le troisième groupe comprend des PLA2 toxiques et fortement basiques ; on les trouve dans un certain nombre de venins de serpents, mais leur rôle et leur mécanisme d'action étaient jusqu'à présent peu connus ; la PLA2 basique de Naja nigricollis appartient à cette catégorie.

La présente invention s'est en conséquence donné pour but de pourvoir à une protéine, isolée de venin de serpent Naja, qui présente des propriétés thérapeutiques intéressantes vis à vis de l'homme.

C'est aussi un but de l'invention de pourvoir à des fragments de ladite protéine présentant une activité antitumorale tout en n'étant pas toxiques.

C'est encore un but de l'invention de pourvoir à des dérivés de ladite protéine obtenus par traitement de celle-ci par un agent approprié et présentant une activité antitumorale tout en n'étant pas toxiques.

C'est encore un but de l'invention de pourvoir à un procédé d'isolement de ladite protéine.

C'est encore un but de l'invention de pourvoir à un procédé de préparation des dérivés, et/ou des fragments de ladite protéine.

C'est également un but de l'invention d'utiliser lesdits dérivés et/ou fragments dans des compositions pharmaceutiques.

C'est également un but de l'invention d'utiliser lesdits dérivés et/ou fragments comme agents de détection sélective des cellules tumorales.

C'est en outre un but de l'invention de fournir un kit, ou coffret, ou ensemble coordonné, prêt à l'emploi, pour la détection sélective des cellules tumorales.

La présente invention a pour objet une protéine basique du type des phospholipases A2 (PLA2), caractérisée en ce qu'elle comprend 118 amino-acides, en ce que son poids moléculaire est de l'ordre de 13 325 Daltons, en ce que son point isoélectrique est de l'ordre de 8,6, en ce qu'elle répond à la formule I ci-après :

$$\text{Asn}^1\text{-Leu-Tyr-Gln-Phe-Lys-Asn-Met-Ile-His}^{10}\text{-Cys-}$$
$$\text{Thr-Val-Pro-Ser-Arg-Pro-Val-Val-His}^{20}\text{-Phe-Ala-Asp-}$$
$$\text{Tyr-Gly-Cys-Tyr-Cys-Gly-Arg}^{30}\text{-Gly-Gly-Lys-Gly-Thr-}$$
$$\text{Pro-Ile-Asp-Asp-Leu}^{40}\text{-Asp-Arg-Cys-Cys-Gln-Val-His-}$$
$$\text{Asp-Asn-Cys}^{50}\text{-Tyr-Glu-Lys-Ala-Gly-Lys-Met-Gly-Cys-}$$
$$\text{Trp}^{60}\text{-Pro-Tyr-Phe-Thr-Leu-Tyr-Lys-Tyr-Lys-Cys}^{70}\text{-}$$
$$\text{Ser-Lys-Gly-Thr-Leu-Thr-Cys-Asn-Gly-Arg}^{80}\text{-Asn-Gly-}$$
$$\text{Lys-Cys-Ala-Ala-Ala-Val-Cys-Asn}^{90}\text{-Cys-Asp-Leu-Val-}$$
$$\text{Ala-Ala-Asn-Cys-Phe-Ala}^{100}\text{-Gly-Ala-Pro-Tyr-Ile-}$$
$$\text{Asn-Ala-Asn-Tyr-Asn}^{110}\text{-Ile-Asp-Phe-Lys-Lys-Arg-}$$
$$\text{Cys-Gln}^{118}\text{-OH,}$$

et en ce qu'elle présente à la fois une activité enzymatique et une activité cytotoxique.

La masse moléculaire a été déterminée par gelfiltration (PAGE - SDS) en référence à l'alpha-neurotoxine de Naja nigricollis (PM : 6 800), au cytochrome C (PM : 12 500), à l'inhibiteur de trypsine de germe de soja (PM : 21 500) et à la sérum albumine bovine (PM 68 000).

Selon un mode de réalisation avantageux de ladite protéine, elle est isolée de venins de serpents de la famille des Elapidés.

Selon une disposition de ce mode de réalisation, ladite protéine est obtenue à partir du venin d'un serpent *Naja* et plus particulièrement de *Naja nigricollis* ou de *Naja mossambica pallida*.

Selon un autre mode de réalisation avantageux de ladite protéine, elle est obtenue par voie de synthèse ou par clonage.

Les Inventeurs ont mis en évidence que l'action toxique létale de ladite protéine est due à la conjugaison de deux actions, à savoir une action cytotoxique et une action enzymatique.

L'invention a également pour objet les fragments et/ou des dérivés de ladite protéine, présentant une activité cytotoxique identique ou analogue à celle de ladite protéine.

Ces dérivés et/ou fragments, outre qu'ils présentent une activité cytotoxique analogue à celle de la phospholipase A2, ne présentent pas l'activité enzymatique de cette dernière, en sorte qu'ils ne sont pas toxiques, l'une des composantes de la conjugaison d'actions précitée étant absente, lesdits dérivés présentant cependant une activité antitumorale sélective.

Parmi lesdits fragments, elle englobe, entre autres :
- un peptide caractérisé en ce qu'il est constitué d'un fragment de la PLA2 et en ce qu'il présente la séquence en amino-acides suivante, qui correspond aux amino-acides en positions 1 à 8 de ladite protéine :
  -Asn$^1$-Leu-Tyr-Gln-Phe-Lys-Asn-Met$^8$-,
- un peptide caractérisé en ce qu'il est constitué d'un fragment de PLA2 et en ce qu'il présente la séquence en amino-acides suivante, qui correspond aux amino-acides en positions 55-59 de ladite PLA2 :

-Gly$^{55}$-Lys-Met-Gly-Cys$^{59}$-,

- un peptide caractérisé en ce qu'il est constitué d'un fragment de PLA2 et en ce qu'il présente la séquence en amino-acides suivante, qui correspond aux amino-acides en positions 44-50 de ladite PLA2.

-Cys$^{44}$-Gln-Val-His-Asp-Asn-Cys$^{50}$-.

L'invention englobe également les dérivés de ladite protéine, présentant une modification de l'une des histidines présente dans la séquence de ladite protéine, caractérisés en ce qu'ils présentent une activité cytotoxique identique ou analogue à celle de ladite protéine tout en ne présentant pas l'activité enzymatique de celle-ci et en ce qu'ils sont modifiés par fixation sur ladite histidine d'un groupe alkyle de formule R-CH$_2$-X, dans laquelle X est un atome d'halogène, de préférence de brome, et R est un groupe aliphatique ou aromatique comprenant de 1 à 20 atomes de carbone.

L'agent alkylant est avantageusement fixé sur l'histidine en position 47 de la protéine selon l'invention et est de préférence du bromure de bromophénacyle.

La présente invention a également pour objet des conjugués, caractérisés en ce que lesdits protéine; fragments et/ou dérivés, sont couplés à une substance de transport sélectif vers une cible appropriée, notamment vers une cellule tumorale, laquelle substance de transport est choisie dans le groupe qui comprend notamment les hormones, les immunoglobulines polyclonales et les immunoglobulines monoclonales.

La présente invention a également pour objet un procédé d'obtention de la phospholipase A2 (PLA2) selon l'invention, caractérisé en ce que ladite protéine est extraite de venin de serpent de la famille des Elapidés, notamment de serpent Naja, et plus particulièrement de Naja nigricollis ou de Naja mossambica pallida par chromatographie par échange d'ions suivie d'une élution par un sel alcalin d'un acide organique faible, notamment de l'acétate d'ammonium, en ce que les fractions éluées présentant une activité enzymatique, sont soumises à une deuxième chromatographie par échange d'ions, et en ce que la protéine ainsi isolée est soumise à une chromatographie liquide haute performance afin d'être purifiée.

La présente invention a également pour objet un procédé d'obtention de dérivés selon l'invention, caractérisé en ce que dans une première étape, on isole la phospholipase A2 (PLA2), selon le procédé décrit ci-dessus, en ce que dans une seconde étape, l'on met en contact ladite phospholipase A2 avec un agent alkylant de type R-CH$_2$-X, où X est un atome d'halogène, de préférence de brome, et R est un groupe aliphatique ou aromatique comprenant de 1 à 20 atomes de carbone, pour modifier la phospholipase au niveau de l'une des histidines présente dans sa séquence, par fixation de l'agent alkylant sur ladite histidine.

L'agent alkylant se fixe préférentiellement sur l'histidine 47 de ladite PLA2 et est avantageusement du bromure de bromophénacyle.

En variante, les dérivés de la phospholipase A2 sont obtenus par un procédé de génie génétique, caractérisé en ce que le cDNA codant pour ladite protéine est modifié par voie génétique au niveau du fragment 44-50 tel que défini ci-dessus.

La présente invention a également pour objet un procédé d'obtention de fragments selon l'invention, caractérisé en ce que lesdits fragments sont obtenus par coupure protéolytique, notamment trypsique et/ou chymotrypsique.

La présente invention a aussi pour objet une composition thérapeutique qui comprend une quantité active d'au moins un dérivé de la phospholipase A2 et/ou un fragment de celle-ci, couplé ou non à une molécule de transport, comme principe actif, notamment pour le traitement des cancers, lequel dérivé et/ou fragment est éventuellement associé à tout véhicule adapté à son administration.

La présente invention a également pour objet un agent de détection de cellules tumorales, caractérisé en ce qu'il est essentiellement constitué par un dérivé et/ou fragment de la phospholipase A2 obtenu conformément à la présente invention.

La présente invention a en outre pour objet un kit, ou coffret, ou ensemble coordonné, prêt à l'emploi pour la détection de cellules tumorales, caractérisé en ce qu'il comprend :

- une quantite appropriée, éventuellement subdivisée en doses unitaires, de dérivé ou fragment selon l'invention,
- éventuellement une quantité appropriée de tampons, diluants, réactifs, nécessaires à la mise en oeuvre de ladite détection.

Selon un mode de réalisation avantageux dudit kit, le dérivé est la pBp-PLA2, obtenue par fixation de bromure de bromophénacyle sur l'histidine 47 de la phospholipase A2 selon l'invention.

Outre les dispositions qui précèdent, l'invention comprend encore d'autres dispositions, qui ressortiront de la description qui va suivre, qui comprend un exemple de préparation d'un dérivé de phospholipase A2 ainsi qu'un compte-rendu d'expérimentations effectuées pour démontrer l'activité cytotoxique spécifique vis-à-vis des cellules tumorales des dérivés et/ou fragments selon l'invention, ainsi que la bonne tolérance desdits dérivés et/ou fragments en comparaison avec la phospholipase A2 de venin de Naja nigricollis.

Il doit être bien entendu, toutefois, que cet exemple et ce compte-rendu d'expérimentations sont donnés uniquement à titre d'illustration de l'objet de l'invention dont ils ne constituent en aucune manière une limitation.

**Exemple A : Extraction et purification de la PLA2**

Le venin lyophilisé provient de l'Institut Pasteur. 3 g de venin sont dissous dans 15 ml d'eau. Après centrifugation, la solution est déposée sur une résine échangeuse d'ions (Biorex 70, Biorad) à laquelle on applique un gradient linéaire d'acétate d'ammonium entre 0,14 M et 1,4 M. Quatre des 10 fractions ainsi séparées possèdent une activité hydrolytique vis-à-vis de la lécithine de jaune d'oeuf, comme l'indiquent des expériences réalisées au pH-stat selon une méthode décrite précédemment (DENNIS E.A., J.Lipid Res., 1986, 14, 152-159). La plus basique d'entre elles, nommée PLA2, est soumise à une nouvelle chromatographie sur résine Biorex 70 en présence d'un gradient linéaire compris entre 0,60 M et 0,95 M. La PLA2 est enfin soumise à une chromatographie liquide à haute performance en phase inverse sur une colonne butyl à larges pores Nucleosil 5 $\mu$m. L'enzyme est éluée par un gradient d'acétonitrile, dans un mélange eau-acide trifluoroacétique.

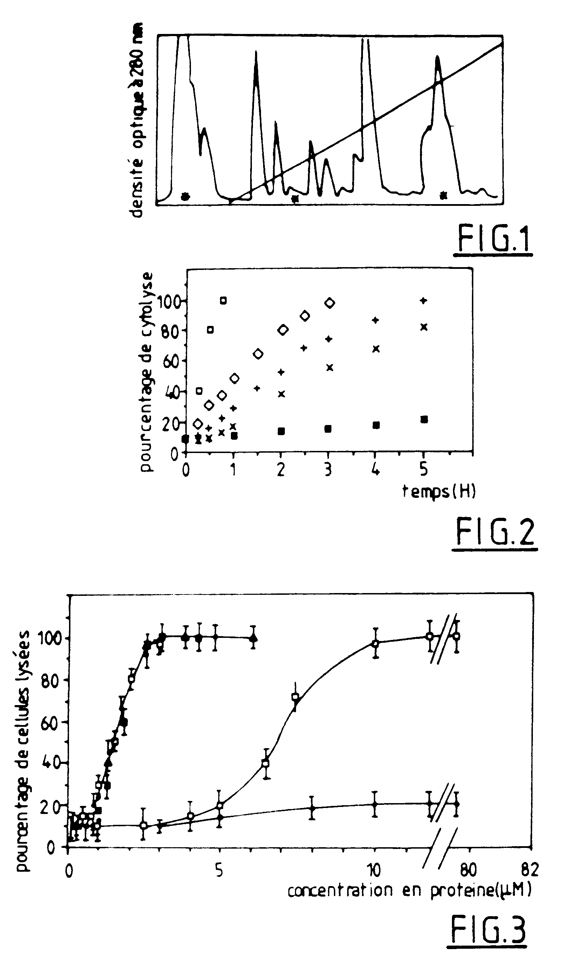

Le profil d'élution est montré sur la figure 1, qui représente le profit d'élution du venin de Naja nigricollis, après chromatographie sur résine échangeuse d'ions Biorex 70. La résine, au préalable, a été équilibrée dans un tampon d'acétate d'ammonium 0,14 M, pH 7,4. Le venin a ensuite été appliqué au sommet d'une colonne (30 x 1 cm). Une fraction, non retenue est éluée immédiatement. Elle renferme une phospholipase A2 comme en témoigne la présence d'un astérisque. Après élution de cette fraction, on applique un gradient d'acétate d'ammonium entre 0,14 M et 1,4 M au même pH. La phospholipase A2 la plus toxique, également indiquée par un astérisque, est éluée dans la dernière fraction, c'est-à-dire la plus à droite sur la figure. C'est cette fraction qui est soumise à une nouvelle chromatographie sur résine Biorex, puis à une HPLC.

**Exemple B :** Préparation de la PLA2 -pBp :

La PLA2 obtenue selon l'exemple A est mise en contact avec du bromure de para bromophénacyle (pBp), dont on sait qu'il interagit préférentiellement sur l'histidine du site actif des phospholipases (VOLWERK et al., Biochem, 1974, 13, 1446-1454).

Les conditions expérimentales de ce traitement sont les suivantes : 150 nmoles de PLA2 sont solubilisées dans 2 ml de tampon cacodylate de sodium (0,1 M), contenant 0,1 M de NaCl, pH 6. On ajoute à cette solution un excès de pBp (5 fois molaire) dans 50 $\mu$l d'acétone. Après 5 heures d'agitation continue dans le noir et à 30°C, le pH du mélange réactionnel est ramené à 3 par addition d'acide acétique glacial. L'échantillon est lyophilisé, puis soumis à une chromatographie liquide à haute performance (colonne butyl à larges pores). Le produit est élué dans un gradient d'hydrophobicité croissante (acétonitrile).

TESTS PHARMACOLOGIQUES

**EXEMPLE 1** : Comparaison des séquences de la PLA2 et de son dérivé pBp.

- Séquence en amino-acides de la PLA2.

. La séquence en amino-acides complète de la phospholipase basique de venin de Naja nigricollis, est déterminée sur la base des peptides obtenus par clivage au moyen de bromure de cyanogène et par protéolyse au moyen de A. Lyticus lysylendopeptidase et S. aureus V8 protéase.

La protéine comprend 118 amino-acides et possède 14 restes $_{1/2}$ cystine.

Le fractionnement de la phospholipase traitée au bromure de cyanogène et carboxyméthylée, fournit 3 peptides de 8, 49 et 61 amino-acides respectivement.

D'autres fragmentations permettent d'obtenir d'autre séquences. Le recouvrement de ces différentes séquences a permis d'établir celle de la PLA2.

- Dérivé pBp.

Le dérivé pBp-PLA2 a perdu une histidine par molécule et son coefficient d'extinction molaire à 276,5 nm est de 45 500.

**EXEMPLE 2** : Toxicité comparée de la PLA2 et de ses dérivés.

La PLA2 et ses dérivés ont été injectés séparément à des souris (18-20g Balb c, femelles) par voie intraveineuse. La PLA2 et ses dérivés sont dissous dans 0,3 ml de soluté physiologique. Une dose de 10 $\mu$g de PLA2 non modifiée est mortelle pour chaque souris. En revanche, une dose de 2,3 mg de PLA2-pBp est non létale pour chaque souris.

### TABLEAU I

| PROTEINES | $DL_{50}$ |
|---|---|
| PLA2 native basique | $5{,}7 \pm 0{,}2{.}10^{-10}$ moles ($7{,}7 \pm 0{,}3\ \mu$g) |
| CNBr - PLA2 | $> 1{,}63{.}10^{-7}$ moles |
| pBp - PLA2 | $> 1{,}60{.}10^{-7}$ moles |

Le tableau I montre les $DL_{50}$ de la PLA2, du dérivé pBp - PLA2 et du dérivé traité au CNBr. La toxicité a été mesurée en injectant les doses indiquées, solubilisées dans 0,3 ml de soluté physiologique, dans la veine de la queue de la souris Balb c (20 ± 1 g). La survie est observée 24 heures après l'injection puis, si nécessaire, chaque jour pendant une semaine au moins. Le signe > signifie, qu'à la dose indiquée, les souris qui ont eu une injection, ne sont pas mortes.

La PLA2 est caractérisée par une activité létale importante. En opposition, les deux dérivés sont non toxiques.

**EXEMPLE 3** : Mesure de l'activité cytotoxique de la PLA2 et de son dérivé pBp.

Les mesures des activités cytotoxiques de la phospholipase PLA2 et de son dérivé pBp s'effectuent de la manière suivante : une suspension de cellules d'une souche homogène définie, contenant $3{,}5{.}10^6$ cellules par ml dans un milieu PBS-DULBECCO, sans calcium et sans magnésium, reçoit des quantités variables de phospholipase ou de son dérivé. L'incubation se poursuit une heure à 37°C. Un colorant, le bleu trypan, est ajouté au milieu. Seules les cellules cytolysées fixent ce colorant. La mesure du nombre de cellules cytolysées permet d'apprécier l'activité cytotoxique du composé étudié. La quantification de cette activité cytotoxique est donnée par la concentration du composé qui tue 50 % des cellules étudiées (AC50), bruit de fond déduit. Le tableau II ci-après montre les concentrations cytotoxiques (AC50%) de la PLA2 et de son dérivé pBp vis-à-vis de huit lignées cellulaires dont cinq sont tumorales et trois sont saines.

| TABLEAU II | | |
|---|---|---|
| SOUCHES CELLULAIRES | PLA2 $AC_{50}(M)$ | PLA2-pBp $AC_{50}(M)$ |
| **Transformées** | | |
| MCF7 (Ho) | $1,5.10^{-6}$ | $3.10^{-5}$ |
| HLGO (Ho) | $3.10^{-6}$ | $5,5.10^{-5}$ |
| SK-N-SH (Ho) | $5.10^{-6}$ | $4.10^{-6}$ |
| BW (Mu) | $3.10^{-6}$ | $> 8.10^{-5}$ |
| C13T (Mu) | $3.10^{-6}$ | $3.10^{-5}$ |
| Normales | | |
| Cellules péritonéales murines | $10^{-5}$ | $> 8.10^{-5}$ |
| Cellules spléniques murines | $10^{-6}$ | $> 4.10^{-5}$ |
| Lymphocytes T murins | $5.10^{-6}$ | $> 8.10^{-5}$ |

Ce tableau indique les concentrations de phospholipase A2 (PLA2) ou modifiée (pBp-PLA2), qu'il est nécessaire de mettre en oeuvre , afin de tuer des cellules transformées ou normales. L'expression > x (où x représente une concentration exprimée en mole/l), indique qu'en présence de ladite concentration de la PLA2 ou de son dérivé, les cellules concernées ne meurent pas.

On voit que la PLA2 naturelle, est cytotoxique pour l'ensemble des souches cellulaires étudiées, qu'elles soient saines ou tumorales. En revanche, le dérivé PLA2-pBp se caractérise par une cytotoxicité sélective pour les cellules tumorales. Quatre des cinq souches transformées sont détruites par cette substance tandis que les trois lignées cellulaires saines ne sont pas altérées.

La figure 2 montre la cinétique de cytolyse des cellules FL, en présence de différentes concentrations de PLA2 de venin de Naja nigricollis ; en abscisse on a le temps en heures, et en ordonnées, on a le pourcentage de cytolyse :

- ▭ correspond à 2 $\mu$M de PLA2
- ◊ correspond à 1 $\mu$M de PLA2
- + correspond à 0,5 $\mu$M de PLA2
- x correspond à 0,25 $\mu$M de PLA2
- ■ correspond à 0 $\mu$M de PLA2

La lyse membranaire des cellules est révélée par un est de pénétration au Bleu Trypan.

Cette figure montre que la PLA2 basique est fortement cytotoxique et que l'effet cytotoxique de la PLA2 dépend à la fois du temps d'incubation et de la concentration en PLA2.

EXEMPLE 4 : Mesure de l'activité enzymatique de la PLA2 et de ses dérivés.

8

L'activité enzymatique correspond à la quantité d'enzyme qui hydrolyse par minute 1 $\mu$mole de phosphatidylcholine à $5,5.10^{-2}$ M, en présence de Triton X100 0,11M à 40°C, pH8. Les valeurs de Vmax sont déterminées dans les mêmes conditions, en utilisant $10^{-8}$ M de PLA2.

Le tableau III ci-après montre que la PLA2 basique présente une activité lipolytique sur la lécithine de jaune d'oeuf. L'activité catalytique est nettement supérieure en présence de Ca++ qu'en présence de Sr++ et n'est pas détectée en présence d'EDTA, même à des concentrations en PLA2 égales à $10^{-6}$ M.

TABLEAU III

|  | Act. Enzymatique (pmoles de PLA2) | Km (mM) | Vmax ($\mu$M NaOH/min.) |
|---|---|---|---|
| PLA2 (Ca$^{++}$) | 20±2 | 7,53±0,20 | 500±14 |
| PLA2 (Sr$^{++}$) | 59±3 | 7,51±0,20 | 170±10 |
| PLA2 (EDTA) | aucune |  |  |
| CNBr-PLA2 (Ca$^{++}$) | 294±11 | 16,30±0,25 | 34±5 |
| pBp-PLA2(Ca$^{++}$) | aucune |  |  |

Le dérivé pBp-PLA2, selon l'invention ne présente pas d'activité enzymatique détectable ; le dérivé CNBr-PLA2 présente une activité catalytique faible mais significative.

La figure 3 montre la lyse de cellules FL après 60 min d'incubation en présence de différentes concentrations de PLA2 (avec ♦6 mM EDTA, ■2mM Sr$^{++}$, ▲2mM Ca$^{++}$) et de dérivés chimiquement traités au bromure de cyanogène (◊) et au bromure de para bromophénacyle (□). Ces expérimentations ont été menées à 37°C, sauf pour ⊞ (2mM Ca$^{++}$ à 4°C). Les autres conditions sont celles de la figure 2.

On a en abscisse, la concentration en protéine en $\mu$M et en ordonnées, le pourcentage de cellules lysées.

Comme le montre cette figure, les courbes doses -réponses obtenues avec la PLA2, en présence ou en absence de calcium, en présence ou en absence de strontium, en présence ou en absence d'EDTA, sont identiques.

On a vérifié que la présence de fortes concentrations de cations divalents ou d'EDTA ne modifie pas la stabilité des cellules pendant la période d'incubation.

La lyse membranaire des cellules est réalisée sur des cellules FL à 37°C ou à 4°C pendant 60 min, mais aucune différence significative n'a été observée pour les courbes à 4°C.

Deux dérivés de la PLA2 ont également été testés ; il s'agit du pBp-PLA2, selon l'invention, obtenu après traitement au bromure de bromophénacyle et du dérivé résultant du clivage de la PLA2 au moyen de bromure de cyanogène en milieu acide.

La figure 3 montre que le dérivé pBp-PLA2 possède une activité cytotoxique vis-à-vis des cellules FL, alors que le dérivé traité au CNBr ne présente pas d'activité cytotoxique.

**Revendications**

**1.** Protéine basique du type des phospholipases A2 (PLA2), caractérisée en ce qu'elle comprend 118 amino-acides, en ce que son poids moléculaire est de l'ordre de 13 325 Daltons, en ce que son point isoélectrique est de l'ordre de 8,6, en ce qu'elle répond à la formule I ci-après :

$$\text{Asn}^1\text{-Leu-Tyr-Gln-Phe-Lys-Asn-Met-Ile-His}^{10}\text{-Cys-}$$
$$\text{Thr-Val-Pro-Ser-Arg-Pro-Val-Val-His}^{20}\text{-Phe-Ala-Asp-}$$
$$\text{Tyr-Gly-Cys-Tyr-Cys-Gly-Arg}^{30}\text{-Gly-Gly-Lys-Gly-Thr-}$$
$$\text{Pro-Ile-Asp-Asp-Leu}^{40}\text{-Asp-Arg-Cys-Cys-Gln-Val-His-}$$
$$\text{Asp-Asn-Cys}^{50}\text{-Tyr-Glu-Lys-Ala-Gly-Lys-Met-Gly-Cys-}$$
$$\text{Trp}^{60}\text{-Pro-Tyr-Phe-Thr-Leu-Tyr-Lys-Tyr-Lys-Cys}^{70}\text{-}$$
$$\text{Ser-Lys-Gly-Thr-Leu-Thr-Cys-Asn-Gly-Arg}^{80}\text{-Asn-Gly-}$$
$$\text{Lys-Cys-Ala-Ala-Ala-Val-Cys-Asn}^{90}\text{-Cys-Asp-Leu-Val-}$$
$$\text{Ala-Ala-Asn-Cys-Phe-Ala}^{100}\text{-Gly-Ala-Pro-Tyr-Ile-}$$
$$\text{Asn-Ala-Asn-Tyr-Asn}^{110}\text{-Ile-Asp-Phe-Lys-Lys-Arg-}$$
$$\text{Cys-Gln}^{118}\text{-OH,}$$

et en ce qu'elle présente à la fois une activité enzymatique et une activité cytotoxique.

2. Protéine selon la revendication 1, caractérisée en ce qu'elle est isolée de venins de serpents de la famille des Elapidés.

3. Protéine selon la revendication 2, caractérisée en ce que en ce qu'elle est obtenue à partir du venin d'un serpent *Naja* et plus particulièrement de *Naja nigricollis* ou de *Naja mossambica pallida.*

4. Protéine selon la revendication 1, caractérisée en ce qu'elle est obtenue par voie de synthèse ou par clonage.

5. Fragment de la protéine selon la revendication 1, caractérisé en ce qu'il est constitué par un peptide présentant la séquence en amino-acides suivante, qui correspond aux amino-acides en positions 1 à 8 de ladite protéine :
-$\text{Asn}^1$-Leu-Tyr-Gln-Phe-Lys-Asn-$\text{Met}^8$-.

6. Fragment de la protéine selon la revendication 1, caractérisé en ce qu'il est constitué par un peptide présentant la séquence en amino-acides suivante, qui correspond aux amino-acides en positions 55-59 de ladite protéine :
-$\text{Gly}^{55}$-Lys-Met-Gly-$\text{Cys}^{59}$-.

7. Fragment de la protéine selon la revendication 1, caractérisé en ce qu'il est constitué par un peptide présentant la séquence en amino-acides suivante, qui correspond aux amino-acides en positions 44-50 de ladite protéine.
-$\text{Cys}^{44}$-Gln-Val-His-Asp-Asn-$\text{Cys}^{50}$-.

8. Dérivés de la protéine selon la revendication 1, présentant une modification de l'une des histidines présente dans la séquence de ladite protéine, caractérisés en ce qu'ils présentent une activité cytotoxique identique ou analogue à celle de ladite protéine tout en ne présentant pas l'activité enzymatique de celle-ci et en ce qu'ils sont modifiés par fixation sur ladite histidine d'un groupe alkyle de formule $R\text{-CH}_2\text{-X}$, dans laquelle X est un atome d'halogène, de préférence de brome, et R est un groupe aliphatique ou aromatique qui comprend de 1 à 20 atomes de carbone.

9. Conjugués, caractérisés en ce que la protéine selon l'une quelconque des revendications 1 à 4, les fragments et/ou dérivés selon l'une quelconque des revendications 5 à 8, sont couplés à une substance de transport sélectif vers une cible appropriée, notamment vers une cellule tumorale, laquelle substance de transport est choisie dans le groupe qui comprend notamment les hormones, les immunoglobulines polyclonales et les immunoglobulines monoclonales.

10. Procédé d'obtention de la phospholipase A2 (PLA2) selon l'une quelconque des revendications 1 à 3, caractérisé en ce que ladite protéine est extraite de venin de serpent de la famille des Elapidés, notamment de serpent *Naja*, et plus particulièrement de *Naja nigricolis* ou de *Naja mossambica pallida* par chromatographie par échange d'ions suivie d'une élution par un sel alcalin d'un acide organique faible, notamment de l'acétate d'ammonium, en ce que les fractions éluées présentant une activité enzymatique sont soumises à une deuxième chromatographie par échange d'ions et ce que la protéine ainsi isolée est soumise à une chromatographie liquide haute performance afin d'être purifiée.

**11.** Procédé d'obtention de dérivés selon la revendication 8, caractérisé en ce que dans une première étape, on isole la phospholipase A2 en mettant en oeuvre le procédé selon la revendication 10, en ce que dans une seconde étape l'on met en contact ladite phospholipase A2 avec un agent alkylant de type $R-CH_2-X$, où X est un atome d'halogène et R est un groupe aliphatique ou aromatique comprenant de 1 à 20 atomes de carbone, pour modifier la phospholipase au niveau de l'une des histidines présente dans sa séquence par fixation de l'agent alkytant sur ladite histidine.

**12.** Procédé d'obtention des dérivés selon la revendication 8, caractérisé en ce que le cDNA codant pour la protéine selon l'une quelconque des revendications 1 à 4 est modifié par voie génétique au niveau du fragment 44-50 de ladite protéine.

**13.** Procédé d'obtention de fragments selon l'une quelconque des revendications 5 à 7, caractérisé en ce que lesdits fragments sont obtenus par coupure protéolytique, notamment trypsique, et/ou chymotrypsique.

**14.** Composition thérapeutique, notamment pour le traitement des cancers, caractérisée en ce qu'elle comprend une quantité active d'au moins un dérivé et/ou d'au moins un fragment, éventuellement couplés à une substance de transport sélectif vers une cible appropriée, notamment vers une cellule tumorale, selon l'une quelconque des revendications 5 à 9, comme principe actif, lequel dérivé et/ou fragment est éventuellement associé à tout véhicule adapté à son administration.

**15.** Agent de détection des cellules tumorales, caractérisé en ce qu'il est essentiellement constitué par un dérivé et/ou un fragment selon l'une quelconque des revendications 5 à 8.

**16.** Kit, ou coffret, ou ensemble coordonné, prêt à l'emploi pour la détection de cellules tumorales, caractérisé en ce qu'il comprend :
- une quantité appropriée, éventuellement subdivisée en doses unitaires, de dérivé et/ou fragment selon l'une quelconque des revendications 5 à 8,
- éventuellement une quantité appropriée de tampons, diluants, réactifs, nécessaires à la mise en oeuvre de ladite détection.

**17.** Kit selon la revendication 16, caractérisé en ce que le dérivé est de la pBp-PLA2, obtenue par fixation de bromure de bromophénacyle sur l'histidine 47 de la protéine selon l'une quelconque des revendications 1 à 4.

**Claims**

**1.** A basic protein of the type of phospholipases A2 (PLA2), characterized in that it comprises 118 amino-acids, has a molecular weight of approximately 13,325 daltons, has an isoelectric point of about 8,6, has the following sequence of amino-acids of formula I below :

$Asn^1$-Leu-Tyr-Gln-Phe-Lys-Asn-Met-Ile-$His^{10}$-Cys-Thr-Val-Pro-Ser-Arg-Pro-Val-
Val-$His^{20}$-Phe-Ala-Asp-Tyr-Gly-Cys-Tyr-Cys-Gly-$Arg^{30}$-Gly-Gly-Lys-Gly-Thr-
Pro-Ile-Asp-Asp-$Leu^{40}$-Asp-Arg-Cys-Cys-Gln-Val-His-Asp-Asn-$Cys^{50}$-Tyr-Glu-
Lys-Ala-Gly-Lys-Met-Gly-Cys-$Trp^{60}$-Pro-Tyr-Phe-Thr-Leu-Tyr-Lys-Tyr-Lys-
$Cys^{70}$-Ser-Lys-Gly-Thr-Leu-Thr-Cys-Asn-Gly-$Arg^{80}$-Asn-Gly-Lys-Cys-Ala-Ala-
Ala-Val-Cys-$Asn^{90}$-Cys-Asp-Leu-Val-Ala-Ala-Asn-Cys-Phe-$Ala^{100}$-Gly-Ala-Pro-
Tyr-Ile-Asn-Ala-Asn-Tyr-$Asn^{110}$-Ile-Asp-Phe-Lys-Lys-Arg-Cys-$Gln^{118}$-OH

and has both an enzymatic activity and cytotoxic activity

2. The protein according to claim 1, characterized in that it is obtained from the venom of a snake of the family *Elapidae.*

3. The protein according to claim 2, characterized in that it is obtained from the venom of a Naja snake and more particularly *Naja nigricollis* or *Naja mossambica pallida.*

4. The protein according to claim 1, characterized in that it is obtained by synthesis or cloning.

5. A fragment of the protein according to claim 1, characterized in that it is constituted by a peptide having the following amino acid sequence, which corresponds to the amino acids in positions 1 to 8 of the said protein :
-$Asn^1$-Leu-Tyr-Gln-Phe-Lys-Asn-$Met^8$-.

6. A fragment of the protein according to claim 1, characterized in that it is constituted by a peptide having the following amino acid sequence, which corresponds to the amino acids in positions 55-59 of the said protein :
-$Gly^{55}$-Lys-Met-Gly-$Cys^{59}$-.

7. A fragment of the protein according to claim 1, characterized in that it is constituted by a peptide having the following amino acid sequence, which corresponds to the amino acids in position 44-50 of the said protein:
-$Cys^{44}$-Gln-Val-His-Asp-Asn-$Cys^{50}$-.

8. Derivatives of the protein according to claim 1, in which one of the histidines present in the sequence of the said protein is modified, characterized in that said derivatives have a cytotoxic activity identical or analogous to that of the said protein without having the enzymatic activity of the latter, and are modified by fixation to the said histidine of an alkyl group of the formula $R-CH_2-X$, in which X is a halogen atom, preferably a bromine atom, and R is an aliphatic or aromatic group containing from 1 to 20 carbon atoms.

9. Conjugates, characterized in that the protein according to any one of claims 1 to 4, the fragments and/or derivatives according to any one of claims 5 to 8 are coupled to a substance for selective transport to an appropriate target, especially to a tumoral cell, which transport substance is selected from the group comprising, in particular, hormones, polyclonal immunoglobulins and monoclonal immunoglobulins.

10. A method of obtaining the phospholipase A2 (PLA2) according to any one of claims 1 to 3, characterized in that said protein is extracted from the venom of a snake of the family *Elapidae*, especially of a Naja snake and more particularly *Naja nigricollis* or *Naja mossambica pallida* by ion exchange chromatography followed by elution with an alkaline salt of a weak organic acid, especially ammonium acetate, the eluted fractions with enzymatic activity being subjected to ion exchange chromatography a second time and the protein isolated in this way then being subjected to high performance liquid chromatography for purification.

11. A method of obtaining derivatives according to claim 8, characterized in that, in a first step, the phospholipase A2 is isolated by carrying out the method according to claim 10, and in a second step, the said phospholipase A2 is brought into contact with an alkylating agent of the type R-CH₂-X, in which X is a halogen atom and R is an aliphatic or aromatic group containing from 1 to 20 carbon atoms, in order to modify the phospholipase at one of the histidines present in its sequence by fixation of the alkylating agent to the said histidine.

12. A method of obtaining the derivatives according to claim 8, characterized in that the cDNA coding for the protein according to any one of claims 1 to 4 is modified genetically at the fragment 44-50 of the said protein.

13. A method of obtaining fragments according to any one of claims 5 to 7, characterized in that the said fragments are obtained by proteolytic scission, especially tryptic and/or chymotryptic scission.

14. A therapeutic composition, especially for the treatment of cancers, characterized in that it comprises an active amount of at least one derivative and/or at least one fragment, which may or may not be coupled to a substance for selective transport to an appropriate target, especially to a tumoral cell, according to any one of claims 5 to 9, as the active principle, the said derivative and/or fragment optionally being associated with any vehicle suitable for its administration.

15. An agent for detecting tumoral cells, characterized in that it is essentially constituted by a derivative and/or fragment according to any one of claims 5 to 8.

16. A ready-to-use kit for detecting tumoral cells, characterized in that it comprises:
- an appropriate amount, optionally subdivided into unit doses, of a derivative and/or fragment according to any one of claims 5 to 8, and optionally
- an appropriate amount of buffers, diluents and reagents necessary for carrying out the said detection.

17. The kit according to claim 16, characterized in that the derivative is pBp-PLA2 obtained by fixation of bromophenacyl bromide to the histidine in position 47 of the protein according to any one of the claims 1 to 4.

**Patentansprüche**

1. Basisches Protein des Phospholipase-A2-yps (PLA2) , **dadurch gekennzeichnet,** daß es 118 Aminosäuren aufweist, daß sein Molekulargewicht in der Größenordnung von 13 325 Dalton liegt, daß sein isoelektrischer Punkt in der Größenordnung von 8,6 liegt, daß es der nachfolgenden Formel I entspricht:

$$\text{Asn}^1\text{-Leu-Tyr-Gln-Phe-Lys-Asn-Met-Ile-His}^{10}\text{-Cys-}$$
$$\text{Thr-Val-Pro-Ser-Arg-Pro-Val-Val-His}^{20}\text{-Phe-Ala-Asp-}$$
$$\text{Tyr-Gly-Cys-Tyr-Cys-Gly-Arg}^{30}\text{-Gly-Gly-Lys-Gly-Thr}$$
$$\text{Pro-Ile-Asp-Asp-Leu}^{40}\text{-Asp-Arg-Cys-Cys-Gln-Val-His-}$$
$$\text{Asp-Asn-Cys}^{50}\text{-Tyr-Glu-Lys-Ala-Gly-Lys-Met-Gly-Cys-}$$
$$\text{Trp}^{60}\text{-Pro-Tyr-Phe-Thr-Leu-Tyr-Lys-Tyr-Lys-Cys}^{70}\text{-}$$
$$\text{Ser-Lys-Gly-Thr-Leu-Thr-Cys-Asn-Gly-Arg}^{80}\text{-Asn-Gly-}$$
$$\text{Lys-Cys-Ala-Ala-Ala-Val-Cys-Asn}^{90}\text{-Cys-Asp-Leu-Val-}$$
$$\text{Ala-Ala-Asn-Cys-Phe-Ala}^{100}\text{-Gly-Ala-Pro-Tyr-Ile-}$$
$$\text{Asn-Ala-Asn-Tyr-Asn}^{110}\text{-Ile-Asp-Phe-Lys-Lys-Arg-}$$
$$\text{Cys-Gln}^{118}\text{-OH,}$$

13

und daß es gleichzeitig eine enzymatische und eine cytotoxische Aktivität zeigt.

2. Protein nach Anspruch 1, dadurch gekennzeichnet, daß es aus den Giften von Schlangen der Familie der Giftnattern (Elapidae) isoliert ist.

3. Protein nach Anspruch 2, **dadurch gekennzeichnet,** daß es aus dem Gift einer Schlange der echten Kobras (Naja) und insbesondere der Schwarzhalskobra (Naja nigricollis) oder der Naja mossambica pallida gewonnen ist.

4. Protein nach Anspruch 1, dadurch gekennzeichnet, daß es auf dem Weg der Synthese oder durch Klonung gewonnen ist.

5. Fragment des Proteins nach Anspruch 1, **dadurch gekennzeichnet,** daß es durch ein Peptid gebildet ist, das die nachstehende Sequenz aus Aminosäuren aufweist, die den Aminosäuren in den Positionen 1 bis 8 des genannten Proteins entsprechen:
-Asn$^1$-Leu-Tyr-Gln-Phe-Lys-Asn-Met$^8$-.

6. Fragment des Proteins nach Anspruch 1, **dadurch gekennzeichnet,** daß es durch ein Peptid gebildet ist, das die nachstehende Sequenz aus Aminosäuren aufweist, die den Aminosäuren in den Positionen 55 bis 59 des genannten Proteins entsprechen:
-Gly$^{55}$-Lys-Met-Gly-Cys$^{59}$-.

7. Fragment des Proteins nach Anspruch 1, **dadurch gekennzeichnet,** daß es durch ein Peptid gebildet ist, das die nachstehende Sequenz aus Aminosäuren aufweist, die den Aminosäuren in den Positionen 44 bis 50 des genannten Proteins entsprechen:
-Cys$^{44}$-Gln-Val-His-Asp-Asn-Cys$^{50}$-.

8. Derivate des Proteins nach Anspruch 1, die eine Modifikation eines der Histidine aufweisen, das in der Sequenz des genannten Proteins vorliegt, **dadurch gekennzeichnet,** daß sie eine cytotoxische Aktivität aufweisen, die identisch oder analog jener des genannten Proteins ist, während sie nicht dessen enzymatische Aktivität aufweisen, und daß sie durch Fixierung einer Alkylgruppe mit der Formel R-CH$_2$-X am genannten Histidin modifiziert sind, in welcher X ein Halogenatom, bevorzugt Brom, und R eine aliphatische oder aromatische Gruppe ist, die 1 bis 20 Kohlenstoffatome umfaßt.

9. Konjugate, **dadurch gekennzeichnet,** daß das Protein nach irgendeinem der Ansprüche 1 bis 4, die Fragmente und/oder Derivate nach irgendeinem der Ansprüche 5 bis 8 an eine Substanz zum selektiven Transport an einen geeigneten Zielort gekoppelt sind, insbesondere an eine Tumorzelle, wobei diese Transportsubstanz in der Gruppe ausgewählt ist, die insbesondere die Hormone, die polyclonalen Immunglobuline und die monoclonalen Immunglobuline umfaßt.

10. Verfahren zur Gewinnung von Phospholipase A2 (PLA2) nach irgendeinem der Ansprüche 1 bis 3, **dadurch gekennzeichnet,** daß das genannte Protein aus dem Gift einer Schlange der Familie der Giftnattern (Elapidae), insbesondere einer Schlange der echten Kobras (Naja) und ganz besonders der Schwarzhalskobra (Naja nigricollis) oder der Naja mossambica pallida durch Ionenaustausch-Chromatographie gewonnen wird, gefolgt von einer Elution durch ein alkalisches Salz einer schwachen organischen Säure, insbesondere Ammoniumazetat, daß die eluierten Fraktionen, die eine enzymatische Aktivität aufweisen, einer zweiten Ionenaustausch-Chromatographie unterworfen werden, und daß das so isolierte Protein zur Reinigung einer Hochleistungs-Flüssigkeitschromatographie mit hoher Leistung unterworfen wird.

11. Verfahren zur Gewinnung von Derivaten nach Anspruch 8, **dadurch gekennzeichnet,** daß man in einem ersten Schritt die Phospholipase A2 isoliert, indem man das Verfahren nach Anspruch 10 durchführt, daß man in einem zweiten Schritt die genannte Phospholipase A2 mit einem Alkylierungsmittel der Typs R-CH$_2$-X in Kontakt bringt, in welchem X ein Halogenatom und R eine aliphatische oder aromatische Gruppe ist, die 1 bis 20 Kohlenstoffatome umfaßt, um die Phospholipase auf der Ebene eines der Histidine zu modifizieren, das in ihrer Sequenz vorliegt, durch Fixierung des Alkylierungsmittels am genannten Histidin.

14

**12.** Verfahren zur Gewinnung von Derivaten nach Anspruch 8, **dadurch gekennzeichnet,** daß die cDNA, die das Protein nach irgendeinem der Ansprüche 1 bis 4 codiert, auf genetischem Weg auf der Ebene des Fragments 44-50 des genannten Proteins modifiziert wird.

**13.** Verfahren zur Gewinnung von Fragmenten nach irgendeinem der Ansprüche 5 bis 7, **dadurch gekennzeichnet,** daß die genannten Fragmente durch proteolytische, insbesondere tryptische, und/oder chymotryptische Spaltung gewonnen werden.

**14.** Therapeutisches Präparat, insbesondere zur Krebsbehandlung, **dadurch gekennzeichnet,** daß es eine aktive Menge mindestens eines Derivats und/oder mindestens eines Fragments, die gegebenenfalls gekoppelt sind an eine Substanz zum selektiven Transport an einen geeigneten Zielort, insbesondere eine Tumorzelle, nach einem der Ansprüche 5 bis 9, als Wirkstoff aufweist, wobei das Derivat und/oder Fragment gegebenenfalls zusammen mit einem Arzneistoffträger vorliegt, der zu seiner Verabreichung geeignet ist.

**15.** Mittel zur Detektion von Tumorzellen, **dadurch gekennzeichnet,** daß es im wesentlichen von einem Derivat und/oder Fragment nach irgendeinem der Ansprüche 5 bis 8 gebildet ist.

**16.** Kit oder Behältnis oder aufeinander abgestimmte Garnitur, das bzw. die vorbereitet ist zur Detektion von Tumorzellen, **dadurch gekennzeichnet,** daß es bzw. sie folgendes enthält:
- eine geeignete, gegebenenfalls in Einheitsdosen aufgeteilte Menge eines Derivats und/oder Fragments nach irgendeinem der Ansprüche 5 bis 8, und
- gegebenenfalls eine geeignete Menge von Puffern, Verdünnungsmitteln, Reagentien, die notwendig sind zur Durchführung der genannten Detektion.

**17.** Kit nach Anspruch 16, **dadurch gekennzeichnet,** daß das Derivat pBp-PLA2 ist, das durch Fixieren des Broms von Bromphenacyl am Histidin 47 des Proteins nach irgendeinem der Ansprüche 1 bis 4 gewonnen worden ist.

FIG.1

FIG.2

FIG.3